# EUROPÄISCHE PATENTSCHRIFT

(11) **EP 1 753 499 B1**
(45) Veröffentlichungstag und Bekanntmachung des Hinweises auf die Patenterteilung: **12.11.2014**
(21) Anmeldenummer: 05757028.5
(22) Anmeldetag: 03.05.2005
(51) Int. Cl.: A61K 9/70, A61K 35/20, A61F 13/00

(54) **AUS DECKFOLIE, VLIESFOLIE, THERAPIEMITTELSCHICHT BESTEHENDE THERAPIEMITTELPACKUNG**
THERAPEUTIC device system comprising a cover foil, a fleece and a layer with a therapeutic principle
Système d'disposiif thérapeutique contenant un film de recouvrement, un non-tissé et une couche avec le principe thérapeutique

(30) Priorität: 05.05.2004 DE 202004007363 U
(43) Veröffentlichungstag der Anmeldung: 21.02.2007
(73) Patentinhaber: Quarkpack GmbH, 94060 Pocking (DE)
(72) Erfinder: LIEBL, Michael, A-4770 Andorf (AT)
(74) Vertreter: Schmidt, Axel
(86) Internationale Anmeldenummer: PCT/DE2005/000832
(87) Internationale Veröffentlichungsnummer: WO 2005/107846

(56) Entgegenhaltungen:
- GB-A- 863 875
- GB-A- 942 466
- GB-A- 1 224 009
- US-A- 6 096 942

## Beschreibung

Die Erfindung bezieht:sich auf eine Therapiemittelpackung für therapeutische Anwendungen mit einer das Therapiemittel umfassenden Umhüllung.

Die Anwendung von Quark als besonders bevorzugtes Beispiel eines derartigen Therapiemittels für therapeutische Zwecke ist seit langem bekannt, beispielsweise zur Heilung oder Linderung von Muskel- oder Gelenkserkrankungen. Hierbei wird das Therapiemittel in ausreichender Menge aus einem Vorratsbehälter entnommen und mit einer bestimmten Stärke von beispielsweise Fingerbreite und vollflächig auf die zu behandelnden Körperteile, z. B. Schenkel oder Arm aufgetragen, und für eine gewisse Behandlungszeit von einigen Minuten, in der die Wirkstoffe des Therapiemittels in die Hautoberfläche einwirken können, belassen. Nach der Behandlung muss die behandelte Körperstelle von Therapiemittelresten gesäubert werden.

GB 942 466, GB 863 875, GB 1 224 009 legen Wundverbände offen, US 6 096 942 bezieht sich auf einen Operationsverband.

US4810499 wie US4927687 offenbaren kleinflächige, Hormone enthaltende Reservoirpflaster zur Wirkstofffreisetzung mit systemischer Wirkung.

Der vorliegenden Erfindung liegt die Aufgabe zugrunde, eine Therapiemittelpackung für therapeutische Anwendungen der gattungsgemäßen Art zur Verfügung zu stellen, welche eine einfachere Handhabung ermöglicht.

Die Lösung der Aufgabe erfolgt durch eine Therapiemittelpackung nach dem Anspruch 1.

Die erfindungsgemäße Therapiemittelpackung für therapeutische Anwendungen hat eine aus einer Deckfolie und einer Vliesfolie bestehende großflächige Umhüllung, in welcher eine Therapiemittelschicht mit vorbestimmter Stärke aufgenommen ist, wobei die Deckfolie und die Vliesfolie an den Rändern miteinander verbunden sind, und die Deckfolie aus einem flüssig- und gasdichten, reißfesten Material und die Vliesfolie aus einem die Wirkstoffe des Therapiemittels durchlässigen, Poren aufweisenden Material besteht.

Mit dem erfindungsgemäßen Gegenstand nach Anspruch 1 wird eine wesentlich einfachere Handhabung ermöglicht. Bei der therapeutischen Anwendung entfällt das Herausnehmen des Therapiemittels aus der Umhüllung und das mühevolle Aufstreichen des Therapiemittels auf die zu behandelnden Körperbereiche. Erfindungsgemäß wird einfach die Therapiemittelpackung auf, die zubehandelnden Körperteile aufgelegt, und zwar mit der die Wirkstoffe des Therapiemittels durchlässigen Seite der Vliesfolie auf die Haut des Patienten. Diese Wirkstoffe des Therapiemittels können während der Behandlungsd'auer in die betreffenden Körperbereiche eindringen und dort ihre medizinische Wirkung entfalten. Nach der Behandlung wird die Therapiemittelpackung einfach vom Körper entfernt und kann entsorgt werden. Eine Säuberung des behandelten Körperbereiches von Therapiemittelresten ist nicht erforderlich, da durch die der Körperhaut zugewandte Vliesfolie lediglich die Wirkstoffe des Therapiemittels und etwas Feuchtigkeit auf die Haut gelangen. Da die Therapiemittelpackung das Therapiemittel in ausreichender Menge in Form einer etwa fingerdicken Therapiemittelpaste oder Therapiemittellage enthält, und zwar in einer vergleichsweise großflächigen Schicht, kann eine Dosierung des aufzutragenden Therapiemittels entfallen. Die Handhabung insbesondere an abgewinkelten Körperteilen wie Knieoder Armgelenk ist mit der erfindungsgemäßen Therapiemittelpackung ebenfalls vereinfacht, da die Therapiemittelpackung lediglich um das Gelenk zu schlagen ist.

Nach einer bevorzugten Weiterbildung der Erfindung ist vorgesehen, dass die Vliesfolie durch eine abziehbare luftdichte Schutzfolie überdeckt ist, die erst unmittelbar vor der Behandlung abgezogen wird. Auf diese Weise wird eine längere Haltbarkeit des Therapiemittels ermöglicht, wobei bei einer gekühlten Lagerung der Therapiemittelpackung diese ohne weiteres für einige Monate frisch bleiben kann. Im übrigen kann eine (oder auch mehrere) einzelne Therapiemittelpackungen des erfindungsgemäßen Gegenstandes problemlos für unterwegs mitgenommen werden.

Dem Prinzip der Erfindung folgend besteht die Vliesfolie aus einem kleinporigem Material, welches lediglich für die über die Feuchtigkeit der Therapiemittelschicht transportierten Wirkstoffe der Therapiemittelschicht durchlässig ist. Insbesondere besteht die Vliesfolie aus einem Viskosevlies, insbesondere reinem Viskosevlies, oder aus einem Zellulose-, Papier- oder Stärkematerial.

Bei einer bevorzugten Weiterbildung der Erfindung weist das Therapiemittel ein kohlenhydrat- oder proteinhaltiges Nahrungsmittel in feuchter oder/und in trockener Form auf, insbesondere Quark, und/oder Kartoffel und/oder Kaffee in feuchter oder/und in trockener Form. Die trockene Form des Therapiemittels umfasst insbesondere auch eine solche in Pulverform, so dass die Therapiepackung nach der Erfindung insbesondere auch mit Quark-, Kartoffel- und/oder Kaffeepulver befüllt sein kann.

Je nach therapeutischem Anwendungsgebiet kann die Therapiemittelschicht mit natürlichen Kräutern und/oder natürlichen oder reinen ätherischen Ölen und/oder anderen natürlichen, medizinisch wirkenden Extrakten angereichert sein. Aus kosmetischen Gründen kann die Therapiemittelschicht darüber hinaus mit Duftstoffen, insbesondere natürlichen Duftölen oder einer Duftkomposition angereichert sein. Für die Anwendung der heilenden Wirkungen der ätherischen Öle ist die Therapiemittelpackung mit den darin enthaltenen Wirkstoffen einfach auf die betreffenden Stellen der Körperoberfläche zu legen, wodurch die Wirkstoffe des Therapiemittels und ggf. ätherischen Öle usw. aufgrund der in dem Therapiemittel gegebenen Feuchtigkeit freigesetzt werden. Weitere Anwendungen der erfindungsgemäßen Therapiemittelpackung etwa in der Aromatherapie sind durchaus beabsichtigt. Die in der erfindungsgemäßen Therapiemittelpackung ggf. enthaltenen bevorzugten duftenden Heiloder Naturkräuter entsprechen dem hohen Standart des deutschen Arzneibuches und sind demzufolge frei von Rückständen oder zumindest rückstandskontrolliert. Die in der Therapiemittelpackung ggf. vorhandenen Duftkomponenten können hierbei aus verschiedenen ätherischen Ölen bestehen. Beispielsweise gibt es bei Kamille kein reines Kamillenöl, welches bezahlbar wäre. Vielmehr werden hervorragende Mischungskomponenten verwendet, die stabil und gut duftend sind.

Bei einer bevorzugten Ausführung der Erfindung hat die die Therapiemittelschicht umhüllende Packung eine Breite von etwa 25 bis 35 cm, insbesondere etwa 28 cm, und eine Länge von 35 cm bis 45 cm, insbesondere etwa 40 cm aufweist.

Bei einer weiteren Ausführung kann vorgesehen sein, dass der Beutel mehrteilig ausgebildet ist oder aus mehreren, untereinander getrennten Kammern besteht. Hierbei kann eine Kammer die Heil- oder Naturkräuter und/oder aromatisierende Extrakte in getrockneter bzw. fester Form, und die andere(n) Kammer(n) die ätherischen Öle und/oder die Farbstoffe und/oder Duftstoffe bzw. deren Komponenten oder Zusammensetzungen enthalten.

Von weiterem Vorteil kann vorgesehen sein, dass die Therapiemittelpackung einzeln in einer luftdichten Deckfolie und abziehbaren Schutzfolie aus jeweils durchsichtigem oder wenigstens durchscheinenden Material verpackt ist. Jede einzelne Therapiemittelpackung nach der Erfindung ist somit von Vorteil beispielsweise in einer beschichteten Polypropylenfolie einzelverpackt. Gegenüber Folien aus anderen Materialien, etwa Papier, Aluminium, oder Polyethylen besteht neben dem Preisvorteil vor allem auch eine wesentlich verbesserte Aromadichtigkeit bei PP-Folien. Außerdem ist die Entsorgungsproblematik geringer; der Wirkstoff Polypropylen kann problemlos im dualen System oder jedem anderen Recyclingsystem entsorgt werden, und ist als Einkomponentenverpackung im Hinblick auf eine Packmittelabgabe zu bevorzugen.

Aufgrund der Durchsichtigkeit der Verpackung wird dem Verbraucher ein direkter Blick auf das Produkt gestattet, so wie er es auch von Produkten aus der Lebensmittelbranche kennt.

In Weiterbildung der Erfindung kann vorgesehen sein, dass die äußere Formgebung der Therapiemittelpackung der äußeren Form des zu behandelnden Körperteiles des Patienten angepasst ist, beispielsweise Gliedmaßen wie Armen und Beinen. Hierbei kann die erfindungsgemäße Therapiemittelpackung des weiteren Befestigungsmittel aufweisen zur Befestigung der Therapiemittelpackung an oder mit dem zu behandelnden Körperteil des Patienten. Ein solches Befestigungsmittel kann beispielsweise einen Klettverschluss oder dergleichen Verschluss- oder Verbindungsmittel besitzen.

Die erfindungsgemäße Therapiemittelpackung kommt insbesondere bei medizinischen oder kosmetischen Indikationen beim Menschen, unter Umständen auch bei Tieren, zur Anwendung. Von der Vielzahl erfindungsgemäßer Verwendungen seien hervorgehoben die entzüdungshemmenden Anwendungen, beispielsweise zur Vermeidung oder Linderung von Brustwarzenentzüdungen, wie sie häufig bei stillenden Müttern vorkommt. Bei einer solchen Anwendung kann die Therapiemittelpackung nach der Erfindung , eine der Brustform angepasste äußere Formgebung haben, so dass diese nach Art eines Brustwickels zur Anwendung bei Brustwarzenentzüdung oder zur Linderung der Schmerzen, wenn die Milch nach der Geburt des Kindes in die Brust der Frau schießt, dienen kann. Eine andere, kosmetische Anwendung betrifft die Straffung der Haut oder Beseitigung von Schwangerschaftsstreifen, oder auch medizinische Anwendungen betreffend Hauterkrankungen wie beispielsweise Cellulite.

Bei einer, weiteren vorteilhaften Ausbildung der Erfindung kann die Therapiemittelpackung desweiteren Füllstoffe als Ergänzungs- und Beimischungsstoffe zur Erhöhung der mechanischen Stabilität der Packung aufweisen, z. B. Zellstoffe, Kunststoffe, Verbundmaterialien, oder auch organische Stoffe wie Obst und Gemüse etc.

Weitere Vorteile der Erfindung ergeben sich aus den Unteransprüchen.

Weitere Zweckmäßigkeiten, Vorteile und Merkmale der Erfindung ergeben sich aus der Beschreibung von bevorzugten Ausführungsbeispielen anhand der Zeichnung. Es zeigt:
- Figur 1: ein bevorzugtes Ausführungsbeispiel der erfindungsgemäßen Therapiemittelpackung,
- Figur 2: ein weiteres Ausführungsbeispiel der erfindungsgemäßen Therapiemittelpackung,
- Figur 3: ein weiteres Ausführungsbeispiel der erfindungsgemäßen Therapiemittelpackung als Brustwickelpackung, und
- Figur 4: ein weiteres Ausführungsbeispiel der erfindungsgemäßen Therapiemittelpackung als Gesichtswickelpackung.

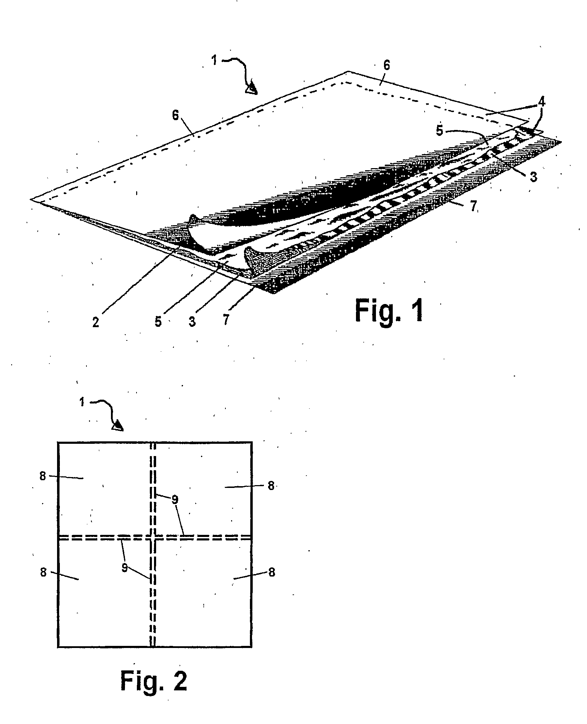

Die in den Figuren 1 bis 4 schematisch dargestellten Ausführungsbeispiele einer erfindungsgemäßen Therapiemittelpackung 1 für therapeutische Anwendungen haben eine aus einer Deckfolie 2 und einer Vliesfolie 3 bestehende großflächige Umhüllung 4, in welcher eine Therapiemittelschicht 5 (z. B. Therapiemittelpaste) mit vorbestimmter Stärke, z. B. fingerdick (etwa 1 cm) aufgenommen ist, wobei die Deckfolie 2 und die Vliesfolie 3 zumindest an den Rändern 6 miteinander verbunden, z. B. verschweisst sind, und die Deckfolie 2 aus einem flüssig- und gasdichten, reißfesten Material und die Vliesfolie aus einem die Wirkstoffe des Therapiemittels 5 durchlässigen, Poren aufweisenden Materials besteht. Insbesondere besteht die Vliesfolie 3 aus einem kleinporigem Material, welches lediglich für die über die Feuchtigkeit der Therapiemittelschicht 5 transportierten Wirkstoffe der Therapiemittelschicht durchlässig ist. Vorzugsweise besteht die Vliesfolie aus einem Viskosevlies, insbesondere reinem Viskosevlies, oder aus einem Zellulose-, Papier- oder Stärkematerial. Die Vliesfolie 3 ist durch eine luftdichte Schutzfolie 7 überdeckt, die vor dem Gebrauch der Packung 1 einfach abgezogen wird.

Bei dem bevorzugten Ausführungsbeispiel nach Figur 1 hat die die Therapiemittelschicht 5 umhüllende Packung 1 eine Breite b von etwa 25 bis 35 cm , insbesondere etwa 28 cm, und eine Länge 1 von 35 cm bis 45 cm, insbesondere etwa 40 cm.

Bei dem in Figur 2 dargestellten Ausführungsbeispiel ist die die Therapiemittelschicht 5 umhüllende Packung 1 mehrteilig ausgebildet und besteht aus mehreren, untereinander getrennten Kammern 8, an deren durch gestrichelte Linien angedeuteten Rändern 9 die Deckfolie 2 und die Vliesfolie 3 miteinander verbunden, z. B. verschweisst sind. Diese Kammern 8 bzw. Sektionen können wie in Figur 2 dargestellt eine rechteckige oder quadratische Grundform, oder aber auch eine beliebig andere (nicht näher dargestellte) z. B. dreieckige oder mehreckige Grundform aufweisen.

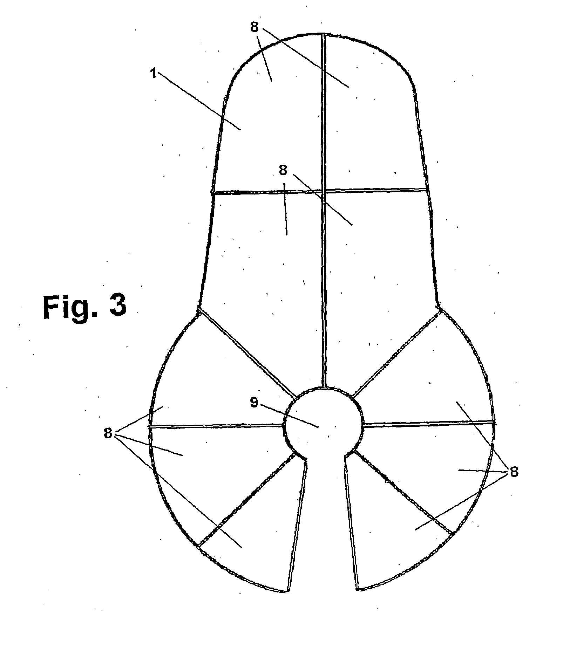

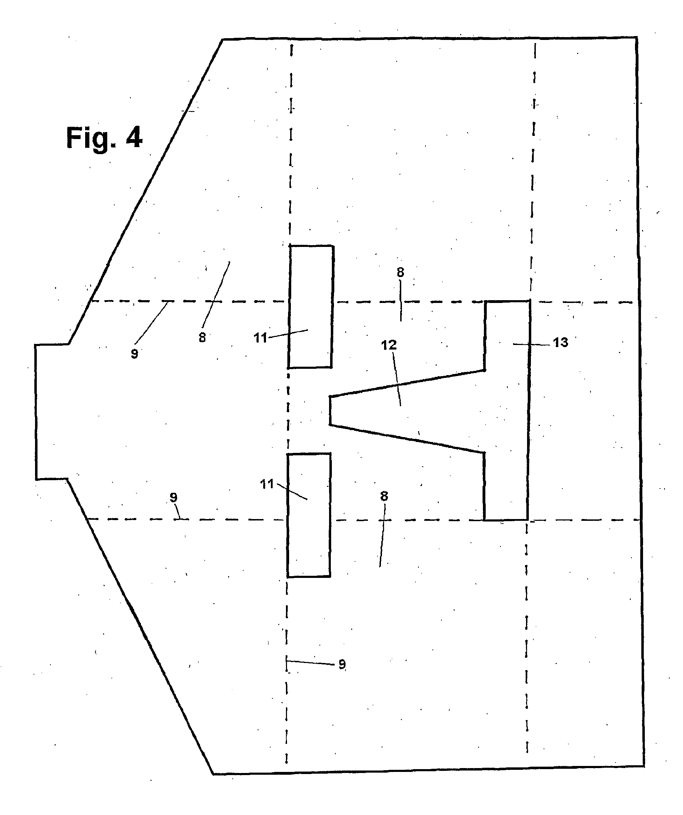

Durch diese Unterteilung der Packung 1 in mehrere Kammern 8 kann die erfindungsgemäße Therapiemittelpackung 1 besser an die äußere Form des zu behandelnden Körperteiles des Patienten angepasst werden, wobei die äußere Formgebung bzw. die Grundform der erfindungsgemäßen Therapiemittelpackung der äußeren Form des zu behandelnden Körperteiles des Patienten angepasst ist. Bei den in Figuren 3 und 4 dargestellten Aüsführungsbeispiele hat die die Therapiemittelschicht 5 umhüllende Packung 1 eine an die äußere Form des zu behandelnden Körperteiles des Patienten angepasste Grundform, und zwar zeigt Figur 3 eine Therapiemittelpackung 1 als Brustwickelpackung, welche eine Durchtrittsöffnung 10 für den Brustwarzenbereich hat, und Figur 4 eine Therapiemittelpackung 1 als Gesichtswickelpackung, welche Durchtrittsöffnungen 11, 12 und 13 für die Augen-, Nasen- und Mundpartien hat.

Bei allen Ausführungsbeispielen der Erfindung kann die Therapiemittelpackung 1 ein (nicht näher dargestelltes) Befestigungsmittel zur Befestigung der Therapiemittelpackung an oder mit dem zu behandelnden Körperteil des Patienten aufweisen, z. B. mittels Klettverschluss zu verbindende Bandabschnitte oder dergleichen.

## Patentansprüche

1. Therapiemittelpackung für therapeutische Anwendungen mit einer aus einer Deckfolie (2) und einer Vliesfolie (3) bestehenden großflächigen Umhüllung (4), in welcher ein Therapiemittel in Form eines kohlenhydrat- oder proteinhaltiges Nahrungsmittels in feuchter oder/und in trockener Form als Therapiemittelschicht (5) mit vorbestimmter Stärke aufgenommen ist,
wobei die Deckfolie (2) und die Vliesfolie (3) an den Rändern (6) miteinander verbunden sind, und
die Deckfolie (2) aus einem flüssig- und gasdichten, reißfesten Material und
die Vliesfolie (3) aus einem die Wirkstoffe des Therapiemittels (5) durchlässigen, Poren aufweisenden Material besteht.

2. Therapiemittelpackung nach Anspruch 1, **dadurch gekennzeichnet, dass** die Vliesfolie (3) aus einem kleinporigem Material besteht, welches lediglich für die über die Feuchtigkeit der Therapiemittelschicht transportierten Wirkstoffe der Therapiemittelschicht (5), durchlässig ist.

3. Therapiemittelpackung nach Anspruch 1 oder 2, **dadurch gekennzeichnet, dass** die Vliesfolie (3) aus einem Viskosevlies, reinem Viskosevlies, oder aus einem Zellulose-, Papier- oder Stärkematerial besteht.

4. Therapiemittelpackung nach einem der vorhergehenden Ansprüche, **dadurch gekennzeichnet, dass** das Therapiemittel Quark, und/oder Kartoffel und/oder Kaffee in feuchter oder/und in trockener Form aufweist.

5. Therapiemittelpackung nach Anspruch 1, 2 oder 3, **dadurch gekennzeichnet, dass** die Therapiemittelschicht (5) mit natürlichen Kräutern und/oder natürlichen oder reinen ätherischen Ölen und/oder anderen natürlichen, medizinisch wirkenden Extrakten angereichert ist.

6. Therapiemittelpackung nach einem der vorhergehenden Ansprüche, **dadurch gekennzeichnet, dass** die Therapiemittelschicht (5) mit Duftstoffen, natürlichen Duftölen oder einer Duftkomposition angereichert ist.

7. Therapiemittelpackung nach einem der vorhergehenden Ansprüche, **dadurch gekennzeichnet, dass** die die Therapiemittelschicht (5) umhüllende Packung (1) eine Breite von etwa 25 bis 35 cm, etwa 28 cm, und eine Länge von 35 cm bis 45 cm, etwa 40 cm aufweist.

8. Therapiemittelpackung nach einem der vorhergehenden Ansprüche, **dadurch gekennzeichnet, dass** die Vliesfolie (3) durch eine abziehbare luftdichte Schutzfolie (7) überdeckt ist.

9. Therapiemittelpackung nach einem der vorhergehenden Ansprüche, **dadurch gekennzeichnet, dass** die Umhüllung (4) mehrteilig ausgebildet ist oder aus mehreren, untereinander getrennten Kammern (8) besteht.

10. Therapiemittelpackung nach einem der vorhergehenden Ansprüche, **dadurch gekennzeichnet, dass** die äußere Formgebung der Therapiemittelpackung der äußeren Form des zu behandelnden Körperteiles des Patienten angepasst ist.

11. Therapiemittelpackung nach einem der vorhergehenden Ansprüche, **dadurch gekennzeichnet, dass** sie Befestigungsmittel zur Befestigung der Therapiemittelpackung an oder mit dem zu behandelnden Körperteil des Patienten aufweist.

## Claims

1. A therapeutic substance package for therapeutic applications, having a large-area envelope (4), which is composed of a covering film (2) and a fleece film (3) and serves to contain a therapeutic substance in the form of a carbohydrate- or protein-containing foodstuff in a wet and/or dry form, embodied as a therapeutic substance layer (5) with a predetermined thickness,
wherein the covering film (2) and the fleece film (3) are connected to each other at the edges (6),
the covering film (2) is composed of a fluid-tight and gas-tight, tear-resistant material, and
the fleece film (3) is composed of a porous material that is penetrable by the active ingredients of the therapeutic substance (5).

2. The therapeutic substance package according to claim 1, **characterized in that** the fleece film (3) is composed of a finely porous material that is penetrable only by the active ingredients of the therapeutic substance layer (5) that are transported via the moisture of the therapeutic substance layer.

3. The therapeutic substance package according to claim 1 or 2, **characterized in that** the fleece film (3) is composed of a viscose fleece, of pure viscose fleece, or of a cellulose, paper, or starch material.

4. The therapeutic substance package according to one of the preceding claims, **characterized in that** the therapeutic substance contains quark and/or potato and/or coffee in a wet and/or dry form.

5. The therapeutic substance package according to claim 1, 2, or 3, **characterized in that** the therapeutic substance layer (5) is enriched with natural herbs and/or natural or pure essential oils and/or other natural, pharmaceutically active extracts.

6. The therapeutic substance package according to one of the preceding claims, **characterized in that** the therapeutic substance layer (5) is enriched with fragrances, natural aromatic oils, or a fragrance composition.

7. The therapeutic substance package according to one of the preceding claims, **characterized in that** the package (1) enveloping the therapeutic substance layer (5) has a width of approximately 25 to 35 cm, for example 28 cm, and a length of 35 cm to 45 cm, for example 40 cm.

8. The therapeutic substance package according to one of the preceding claims, **characterized in that** the fleece film (3) is covered with a removable airtight protective film (7).

9. The therapeutic substance package according to one of the preceding claims, **characterized in that** the envelope (4) is composed of multiple parts or is composed of a plurality of chambers (8) that are separate from one another.

10. The therapeutic substance package according to one of the preceding claims, **characterized in that** the outer form of the therapeutic substance package is adapted to the outer form of the part of the patient's body to be treated.

11. The therapeutic substance package according to one of the preceding claims, **characterized in that** it has fastening means for fastening the therapeutic substance package on or to the part of the patient's body to be treated.

## Revendications

1. Emballage d'agent thérapeutique destiné à des applications thérapeutiques, comportant une enveloppe de grande surface (4) composée d'une pellicule de couverture (2) et d'une pellicule de non-tissé (3), contenant un agent thérapeutique sous la forme d'un agent alimentaire contenant des hydrocarbures ou des protéines sous forme humide et/ou sèche à titre de couche d'agent thérapeutique (5) d'épaisseur prédéfinie,
la pellicule de couverture (2) et la pellicule de non-tissé (3) étant reliées sur les bords (6), et
la pellicule de couverture (2) étant composée d'un matériau étanche aux liquides et aux gaz, résistant à la déchirure, et
la pellicule de non-tissé (3) étant composée d'un matériau poreux perméable aux principes actifs de l'agent thérapeutique (5).

2. Emballage d'agent thérapeutique selon la revendication 1, **caractérisé en ce que** la pellicule de non-tissé (3) est composée d'un matériau à petits pores qui est perméable uniquement aux principes actifs de la couche d'agent thérapeutique (5) transportés par l'humidité de la couche d'agent thérapeutique.

3. Emballage d'agent thérapeutique selon la revendication 1 ou 2, **caractérisé en ce que** la pellicule de non-tissé (3) est composée d'un non-tissé à base de viscose, de non-tissé de viscose pur ou d'un matériau à base de cellulose, de papier ou d'amidon.

4. Emballage d'agent thérapeutique selon l'une des revendications précédentes, **caractérisé en ce que** l'agent thérapeutique comprend du fromage blanc et/ou de la pomme de terre et/ou du café sous forme humide et/ou sèche.

5. Emballage d'agent thérapeutique selon la revendication 1, 2 ou 3, **caractérisé en ce que** la couche d'agent thérapeutique (5) est enrichie d'herbes naturelles et/ou d'huiles essentielles naturelles ou pures et/ou d'autres extraits naturels à effet médical.

6. Emballage d'agent thérapeutique selon l'une des revendications précédentes, **caractérisé en ce que** la couche d'agent thérapeutique (5) est enrichie de parfums, d'huiles parfumées naturelles ou d'une composition parfumée.

7. Emballage d'agent thérapeutique selon l'une des revendications précédentes, **caractérisé en ce que** l'emballage (1) enveloppant la couche d'agent thérapeutique (5) présente une largeur d'environ 25 à 35 cm, d'environ 28 cm, et une longueur de 35 cm à 45 cm, d'environ 40 cm.

8. Emballage d'agent thérapeutique selon l'une des revendications précédentes, **caractérisé en ce que** la pellicule de non-tissé (3) est recouverte par une pellicule de protection pelable, étanche à l'air (7).

9. Emballage d'agent thérapeutique selon l'une des revendications précédentes, **caractérisé en ce que** l'enveloppe (4) est réalisée en plusieurs parties ou est composée de plusieurs chambres (8) séparées entre elles.

10. Emballage d'agent thérapeutique selon l'une des revendications précédentes, **caractérisé en ce que** la forme extérieure de l'emballage d'agent thérapeutique est adaptée à la forme extérieure de la partie du corps du patient à traiter.

11. Emballage d'agent thérapeutique selon l'une des revendications précédentes, **caractérisé en ce qu'**il comprend des moyens de fixation destinés à fixer l'emballage d'agent thérapeutique sur ou avec la partie du corps du patient à traiter.
